# FASCICULE DE BREVET EUROPEEN

(11) **EP 0 780 366 B1**
(45) Date de publication et mention de la délivrance du brevet: **26.01.2000**
(21) Numéro de dépôt: 96402538.1
(22) Date de dépôt: 26.11.1996
(51) Int. Cl.: C07C 291/02, C07C 239/08

(54) **Procédé d'obtention d'hydroxylamine N-monosubstituée**
Verfahren zur Herstellung von N-monosubstituierten Hydroxylaminen
Process for the preparation of N-monosubstituted hydroxylamine

(30) Priorité: 19.12.1995 FR 9515040
(43) Date de publication de la demande: 25.06.1997
(73) Titulaire: ELF ATOCHEM S.A., 92800 Puteaux, Hauts-de-Seine (FR)
(72) Inventeur: Calais, Christophe, 69330 Meyzieu (FR); Teissier, Rémy, 69340 Francheville (FR)

(56) Documents cités:
- EP-A- 0 498 346
- FR-A- 2 632 638
- GB-A- 2 032 422
- GB-A- 2 136 422
- US-A- 4 960 934
- D. KLAMANN, H. HAGEMANN: 'Houben-Weyl Methoden der Organischen Chemie', 1990, GEORG THIEME VERLAG, STUTTGART (DE) XP002011416 vol. E14b/2 * page 1409 - page 1415 * * page 1416, alinéa 2 *
- E. MÜLLER: 'Houben-Weyl Methoden der Organischen Chemie', 1968, GEORG THIEME VERLAG, STUTTGART (DE) XP002011417 4eme Ed., vol. X/4, page 444 - page 445

## Description

La présente invention a pour objet un nouveau procédé d'obtention de nitrones. Plus particulièrement, elle concerne un procédé d'obtention d'hydroxylamine N-monosubstituée comprenant une étape dans laquelle une nitrone est formée à partir d'une amine secondaire.

Les alkylhydroxylamines sont connues en tant que réducteurs et capteurs de radicaux libres . Elles sont utilisées comme inhibiteurs de polymérisation ou inhibiteurs de corrosion dans le traitement des eaux de chaudière. Par rapport à des inhibiteurs de corrosion classiques tels que l'hydrazine ou le sulfite de sodium, les alkylhydroxylamines présentent l'avantage de pouvoir protéger non seulement le bouilleur mais également le système de condensation. Ainsi, la totalité de l'installation de traitement des eaux de chaudière est protégée contre la corrosion.

La synthèse des alkylhydroxylamines N-monosubstituées peut être réalisée selon différentes méthodes. Dans le brevet EP 147 879 est décrit un procédé d'obtention de ces alkylhydroxylamines par réduction des nitroalcanes en présence d'un catalyseur d'hydrogénation à base de platine et d'additifs tels que les bases azotées et les composés organiques du phosphore trivalent ou pentavalent. Ces alkylhydroxylamines sont cependant instables au stockage (EP 321 219) et leur procédé d'obtention à partir des nitroalcanes présente l'inconvénient d'être peu sélectif.

Par ailleurs il est connu que l'hydrolyse acide des nitrones peut conduire aux alkylhydroxylamines N-monosustituées.

Les nitrones sont des intermédiaires de synthèse importants et d'excellents capteurs de radicaux libres. Jusqu'à présent, tous les procédés d'obtention de nitrones à partir des amines secondaires font appel à des catalyseurs à base de métaux de transition. Ainsi, dans J. Chem. Soc. Chem. Commun p 874 (1984), H. MITSUI et al. utilisent une solution aqueuse d'eau oxygénée à 30 % et du tungstate de sodium pour oxyder les amines secondaires en nitrones à 0° C sous argon . Le dichlorométhane est ensuite employé comme solvant pour extraire les nitrones du milieu réactionnel.

Shun-lchi MURAHASHI et al. (J. Org. Chem. 1990, 55, 1736-1744) ont obtenu un rendement de 74 % en diisopropyl nitrone par oxydation de la diisopropyl amine avec de l'eau oxygénée en présence du tungstate de sodium. Le solvant de la réaction est le méthanol.

Selon le même auteur (Tetrahedron Letters Vol. 28 No 21 p. 2383-2386), les nitrones peuvent également être obtenues par oxydation des amines secondaires avec de l'eau oxygénée en présence du dioxyde de sélénium. Ainsi, l'oxydation de la diisopropylamine dans le méthanol conduit à la diisopropyl nitrone avec un rendement de 66 %.

Les hétéropolyoxométallates tels que les peroxotungstophosphates (PCWP) ont également été employés pour oxyder les amines secondaires en nitrones. Dans ce cas, la température est de 0° C et le solvant est le chloroforme (S. SAKAVE et al. dans Chemistry Letters p. 289-292 (1992)).

Selon E. MARCANTONI et al. dans Tetrahedron Letters Vol. 36 No 20 p. 3561-3562 (1995), les amines secondaires peuvent être oxydées en nitrones avec des complexes urée-H₂O₂ (UHP) et en présence de catalyseurs à base de métaux de transition.

Il a maintenant été trouvé un nouveau procédé d'obtention de nitrones à haut rendement , qui présente l'avantage de pouvoir utiliser l'eau comme solvant et qui de plus, ne fait pas intervenir des catalyseurs à base de métaux de transition. Un procédé d'obtention d'hydroxylamine N-monosubstituée à partir d'une amine secondaire et ne présentant pas les inconvénients énoncés précédemment forme également l'objet de la présente invention.

La présente invention présente également l'avantage de conduire à un coproduit carbonylé (aldéhyde ou cétone) valorisable.

La présente invention a donc pour but de fournir un procédé d'obtention de nitrone à partir d'une amine secondaire comprenant au moins un atome d'hydrogène sur au moins un atome de carbone en alpha de l' azote caractérisé en ce que cette amine secondaire est oxydée en présence d'un agent oxydant, et d'au moins un composé choisi parmi le dioxyde de carbone, les hydrogénocarbonates et les carbonates.

Selon l'invention, on peut utiliser une amine secondaire de formule générale 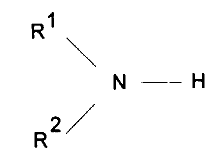 dans laquelle R¹ et R², identiques ou différents, représentent chacun un radical alkyle linéaire, ramifié ou cyclique contenant de 1 à 8 atomes de carbone, comme par exemple les radicaux méthyle, éthyle, propyle, isopropyle, butyle, isobutyle, tertiobutyle, amyle, hexyle ou cyclohexyle, un radical aryle comme par exemple les radicaux phenyle ou tolyle, ou un radical aralkyle comme par exemple le radical benzyle, l'un des groupements R₁ et R₂ devant présenter au moins un atome d'hydrogène sur au moins un atome de carbone en alpha de l'azote.

Les radicaux R¹ et R² peuvent également être reliés entre eux en formant un cycle substitué ou non comme par exemple dans la pyrrolidine, la pipéridine, I' hexaméthylène imine ou l' heptaméthylène imine.

Avantageusement on utilise une amine secondaire non cyclique et de préférence une amine secondaire non cyclique dans laquelle les radicaux R¹ et R² sont identiques.

Les radicaux R¹ et R² particulièrement préférés sont ceux choisis parmi le méthyle, l'éthyle, le propyle, l'isopropyle, le butyle et l'isobutyle.

De préférence on utilise la diisopropylamine.

La transformation d' une amine secondaire cyclique ou non cyclique en nitrone peut être schématisée par l'équation connue ci-dessous:

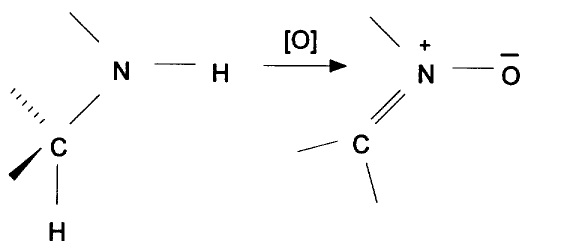

Bien que la formation de nitrone selon la présente invention puisse être effectuée en l'absence de solvant, on opère de préférence en présence d' un solvant. Le solvant peut être soit l'eau soit un solvant organique polaire tel que le méthanol ou l'acétone. La quantité de solvant peut varier dans de larges limites. On utilise généralement une quantité telle que le rapport massique solvant / amine secondaire est compris entre 0,1 et 10 et de préférence compris entre 0,5 et 1. Avantageusement, on utilise l'eau comme solvant.

Lorsqu' on opère en présence d' un solvant, l'amine secondaire peut être introduite dans le réacteur avant, pendant ou après l'introduction de solvant.

La température à laquelle on introduit l'amine secondaire et éventuellement le solvant dans le réacteur, est normalement comprise entre la température ambiante et 70 °C. On préfère introduire l'amine secondaire et le cas échéant le solvant à la température ambiante.

L' agent oxydant utilisé dans le procédé suivant la présente invention peut être choisi parmi les peroxydes, les hydroperoxydes et les peracides. A titre d' exemple on peut citer le peroxyde d' hydrogène, l'hydroperoxyde de tertiobutyle ou l'acide meta-chloroperbenzoïque. Avantageusement, on utilise une solution d'eau oxygénée (peroxyde d'hydrogène) de titre compris entre 5 et 70 % en poids. Des concentrations en eau oxygénée comprises entre 30 et 50 % sont particulièrement préférées.

La quantité d'agent oxydant nécessaire à la transformation d' une amine secondaire en nitrone est en général dans un rapport molaire oxydant / amine secondaire compris entre 1 et 4, de préférence dans un rapport molaire voisin de 2.

Selon la présente invention on utilise l'agent oxydant conjointement avec au moins un composé choisi parmi le dioxyde de carbone, les hydrogénocarbonates et les carbonates. Ce(s) composé(s) est ou sont de préférence introduit(s) dans le milieu réactionnel avant l'agent oxydant.

Parmi les hydrogénocarbonates, on peut citer l'hydrogenocarbonate d'ammonium, l'hydrogénocarbonate d'un métal alcalin tel que le sodium ou le potassium .

Parmi les carbonates, on peut citer le carbonate d' ammonium, le carbonate d' un métal alcalin tel que le sodium ou le potassium.

Ces agents sont généralement utilisés dans un rapport molaire dioxyde de carbone et/ou hydrogénocarbonates et/ou carbonates / amine secondaire compris entre 0,1 et 1 et le plus souvent compris entre 0,1 et 0,3.

Avantageusement le dioxyde de carbone est utilisé conjointement avec l'agent oxydant.

On utilise en général une température comprise entre 20° C et 80° C et de préférence comprise entre 50° C et 70° C pour oxyder l'amine secondaire en nitrone .

Bien que l'oxydation de l'amine secondaire puisse être effectuée à une pression supérieure à la pression atmosphérique, on opère le plus souvent à la pression atmosphérique.

La nitrone obtenue dans le procédé suivant l'invention peut être soit conservée en solution, soit isolée du milieu réactionnel par des méthodes classiques (distillation, extraction ), soit hydrolysée directement en hydroxylamine N-monosubstituée.

L'autre objet de l'invention concerne donc, un procédé d'obtention d'hydroxylamine N-monosubstituée selon lequel la nitrone formée précédemment est hydrolysée.

Le procédé d'obtention d'hydroxylamine N-monosubstituée selon l'invention est caractérisé en ce que l'on soumet à une hydrolyse la nitrone formée à partir d'une amine secondaire, comprenant au moins un atome d'hydrogène sur au moins un atome de carbone en alpha de l'azote, avec l'aide d'un agent oxydant et en présence d' au moins un composé choisi parmi le dioxyde de carbone, les hydrogénocarbonates et les carbonates.

La réaction d' hydrolyse de la nitrone peut être conduite en présence d'un acide minéral ou organique. L'acide minéral est avantageusement choisi parmi l'acide chlorhydrique, l'acide sulfurique, l'acide phosphorique. Comme acide organique on préfère utiliser l'acide acétique ou l'acide oxalique.

L'acide est normalement utilisé dans un rapport molaire H⁺ / amine secondaire compris entre 0,9 et 2 et de préférence voisin de 1.

La température d' hydrolyse est de préférence identique à celle utilisée dans l'étape d'oxydation de l'amine secondaire à savoir une température comprise entre 50 et 70° C.

L' hydrolyse de la nitrone est en général effectuée à une pression inférieure à la pression atmosphérique. On peut soit utiliser une pression réduite constante, soit une pression qui décroît de façon progressive au cours de l'hydrolyse de façon à éliminer le coproduit carbonylé formé dans le cas d' une nitrone non cyclique.

L' hydroxylamine N-monosubstituée formée peut être isolée sous forme de sel par évaporation du solvant sous une pression réduite.

La réaction d' hydrolyse d' une nitrone non cyclique peut être schématisée suivant l'équation ci-dessous :

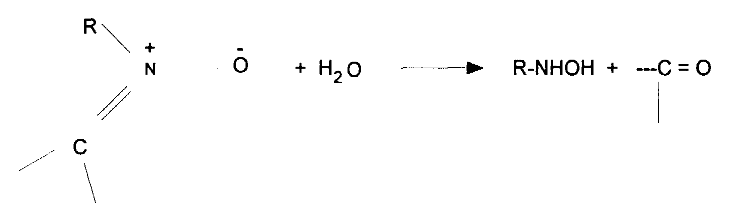

L' hydroxylamine N-monosubstituée particulièrement préférée a pour formule générale : R-NHOH dans laquelle R est un radical méthyle, éthyle, propyle, isopropyle, butyle, isobutyle. Avantageusement l'isopropyl hydroxylamine est obtenue dans le procédé suivant la présente invention.

L' eau nécessaire à la formation de l'hydroxylamine N-monosubstituée peut être ajoutée avant, pendant ou après l'introduction de la nitrone dans le réacteur ou elle peut provenir de l'eau utilisée comme solvant dans l'étape de formation de la nitrone.

Par ailleurs, I' hydrolyse d' une nitrone cyclique conduit à une hydroxylamine N-monosubstituée fonctionnalisée comprenant un groupement carbonylé.

On entend par nitrone cyclique, une nitrone dans laquelle l'atome d'azote forme partie d' un cycle.

L'invention sera mieux comprise à l'aide des exemples suivants :

### Mode opératoire général

On introduit dans un réacteur thermostaté à une température comprise entre 17 et 30° C I' eau suivi de l'amine secondaire à oxyder. On introduit ensuite sous agitation soit le dioxyde de carbone et/ou l'hydrogénocarbonate et/ou le carbonate dans le réacteur. Après addition on porte le mélange réactionnel à une température comprise entre 50 et 70° C en environ 30 minutes. Lorsque le mélange a atteint la température déterminée, on additionne l'agent oxydant en une période allant d' une heure à trois heures.

On suit la transformation de l'amine secondaire et la formation de la nitrone à l'aide de la chromatographie en phase gazeuse ( colonne Chrompack CPWAX 51 CB de 50 m, détecteur FID et analyse en programmation de température par étalonnage interne).

La nitrone formée peut être soit isolée du milieu réactionnel après transformation totale de l'amine secondaire soit soumise directement à l'hydrolyse. Pour l'étape d' hydrolyse on introduit l'acide après consommation totale de l'amine secondaire et ensuite on réduit la pression du milieu réactionnel jusqu' à une valeur comprise entre 300 et 500 mbar . Au bout de 2 à 4 heures, la pression du milieu réactionnel est de nouveau réduite jusqu'à une valeur comprise entre 20 et 150 mbar.

L' hydroxylamine N-monosubstituée est ensuite récupérée sous forme de sel par évaporation sous vide.

### Exemple 1

On introduit à température ambiante dans un réacteur d'un litre thermostaté, 86 g d'eau et 170 g de diisopropylamine (DIPA, pureté 99 %) . 17 g de CO₂ (Air Liquide N45 pureté supérieure à 99,995 %) sont ensuite introduits dans le réacteur. Puis le mélange est chauffé sous agitation. Lorsque la température du mélange a atteint 65° C, on commence l'addition d'une solution aqueuse d'eau oxygénée à 45 %. L'addition des 255 g de la solution aqueuse d'eau oxygénée se fait en deux heures et demie.

Après consommation totale de la DIPA soit environ 4 heures (depuis le début de la réaction), on isole la diisopropylnitrone.

Le rendement en diisopropylnitrone est de 95 %.

### Exemple 2

Cet exemple est réalisé de manière identique à l'exemple 1 sauf que la diisopropylnitrone n' est pas isolée mais soumise directement à l'hydrolyse.

Ainsi après transformation totale de la diisopropylamine on introduit 166 g d'acide chlorhydrique à 37 %. On réduit ensuite la pression dans le réacteur jusqu' à 350 mbar pour permettre la distillation de l'acétone, coproduite lors de la réaction d'hydrolyse. Au bout de trois heures on réduit de nouveau la pression jusqu'à 25 mbar. Après élimination totale d' eau, on récupère 170 g d'hydrochlorure de la N-isopropylhydroxylamine cristallisé. Ceci correspond à un rendement de 90 % par rapport à l'amine de départ.

### Exemple 3

On opère dans les mêmes conditions que celles décrites dans l'exemple 2 sauf qu' on utilise les réactifs suivants :

| | |
|---|---|
| Diéthylamine (DEA) | 125 g |
| Eau | 120 g |
| CO₂ | 18 g |
| H₂O₂ à 45 % | 130 g |
| HCl à 37 % | 150 g |

On obtient l'hydrochlorure de la N-éthylhydroxylamine cristallisé avec un rendement de 72 %.

### Exemple 4

Les conditions opératoires sont les mêmes que dans l'exemple 2, sauf que le réacteur utilisé a une capacité de 250 ml et qu'on a employé divers acides pour l'hydrolyse. Les quantités de réactifs sont les suivantes.

| | |
|---|---|
| DIPA | 0,2 mole (20 g) |
| Eau | 20 g |
| CO₂ | 2 g |
| H₂O₂ 35 % | 20 g |
| Acide | 0,2 équivalent. |

On obtient les sels correspondants de la N-isopropylhydroxylamine (NIPHA) cristallisé avec les rendements suivants :

| | | |
|---|---|---|
| (NIPHA)₂.H₂SO₄ | rendement | 91 % |
| (NIPHA). CH₃COOH | " | 84 % |
| (NIPHA)₃. H₃PO₄ | " | 87 % |
| (NIPHA)₂. H₂C₂O₄ | " | 88 % |

## Revendications

1. Procédé d'obtention de nitrones à partir d'une amine secondaire caractérisé en ce que cette amine secondaire comprenant au moins un atome d' hydrogène sur au moins un atome de carbone en alpha de l'azote est oxydée en présence d'un agent oxydant et d' au moins un composé choisi parmi le dioxyde de carbone, les hydrogénocarbonates et les carbonates.

2. Procédé suivant la revendication 1 caractérisé en ce que l'agent oxydant est le peroxyde d' hydrogène.

3. Procédé suivant la revendications 1 ou 2, caractérisé en ce qu' on opère en présence d' un solvant.

4. Procédé suivant la revendication 3, caractérisé en ce que le solvant est l'eau.

5. Procédé suivant l'une des revendications 1 à 4, caractérisé en ce que l'amine secondaire est la diisopropylamine .

6. Procedé suivant l'une des revendications 1 à 5, caractérisé en ce que la température du milieu réactionnel est comprise entre 20 ° C et 80 ° C.

7. Procédé suivant la revendication 6 caractérisé en ce que la température du milieu réactionnel est comprise entre 50 et 70 °C.

8. Procédé suivant l'une des revendications 1 à 7 caractérisé en ce que le rapport molaire agent oxydant / amine secondaire est compris entre 1 et 4.

9. Procédé suivant l'une des revendications 1 à 8 caractérisé en ce que le rapport molaire dioxyde de carbone et/ou hydrogénocarbonate et/ou carbonate / amine secondaire est compris entre 0,1 et 1.

10. Procédé suivant la revendication 9 caractérisé en ce que le rapport molaire dioxyde de carbone et/ou hydrogénocarbonate et/ou carbonate / amine secondaire est compris entre 0,1 et 0,3.

11. Procédé d'obtention d' hydroxylamine N-monosubstituée, caractérisé en ce que l'on soumet à une hydrolyse la nitrone formée selon l'une des revendications 1 à 10.

12. Procédé suivant la revendication 11, caractérisé en ce que l'hydrolyse de la nitrone s' effectue en présence des acides minéraux ou organiques.

13. Procédé suivant la revendication 12 caractérisé en ce que le rapport molaire H⁺ / amine secondaire est compris entre 0,9 et 2.

## Patentansprüche

1. Verfahren zur Herstellung von Nitronen aus einem sekundären Amin, dadurch gekennzeichnet, daß dieses sekundäre Amin, das mindestens ein Wasserstoffatom an wenigstens einem Kohlenstoffatom in α-Stellung zum Stickstoff enthält, in Gegenwart eines Oxidationsmittels und mindestens einer aus Kohlendioxid, den Hydrogencarbonaten und den Carbonaten ausgewählten Verbindung oxidiert wird.

2. Verfahren nach Anspruch 1, dadurch gekennzeichnet, daß das Oxidationsmittel Wasserstoffperoxid ist.

3. Verfahren nach Anspruch 1 oder 2, dadurch gekennzeichnet, daß man in Gegenwart eines Lösungsmittels arbeitet.

4. Verfahren nach Anspruch 3, dadurch gekennzeichnet, daß das Lösungsmittel Wasser ist.

5. Verfahren nach einem der Ansprüche 1 bis 4, dadurch gekennzeichnet, daß das sekundäre Amin Diisopropylamin ist.

6. Verfahren nach einem der Ansprüche 1 bis 5, dadurch gekennzeichnet, daß die Temperatur des Reaktionsgemisches zwischen 20 °C und 80 °C liegt.

7. Verfahren nach Anspruch 6, dadurch gekennzeichnet, daß die Temperatur des Reaktionsgemisches zwischen 50 °C und 70 °C liegt.

8. Verfahren nach einem der Ansprüche 1 bis 7, dadurch gekennzeichnet, daß das Molverhältnis von Oxidationsmittel zu sekundärem Amin zwischen 1 und 4 liegt.

9. Verfahren nach einem der Ansprüche 1 bis 8, dadurch gekennzeichnet, daß das Molverhältnis von Kohlendioxid und/oder Hydrogencarbonat und/oder Carbonat zu sekundärem Amin zwischen 0,1 und 1 liegt.

10. Verfahren nach Anspruch 9, dadurch gekennzeichnet, daß das Molverhältnis Kohlendioxid und/oder Hydrogencarbonat und/oder Carbonat zu sekundärem Amin zwischen 0,1 und 0,3 liegt.

11. Verfahren zur Herstellung von N-monosubstituiertem Hydroxylamin, dadurch gekennzeichnet, daß man das nach den Ansprüchen 1 bis 10 gebildete Nitron einer Hydrolyse unterzieht.

12. Verfahren nach Anspruch 11, dadurch gekennzeichnet, daß die Hydrolyse des Nitrons in Gegenwart von Mineralsäuren oder organischen Säuren erfolgt.

13. Verfahren nach Anspruch 12, dadurch gekennzeichnet, daß das Molverhältnis von H⁺ zu sekundärem Amin zwischen 0,9 und 2 liegt.

## Claims

1. Process for obtaining nitrones from a secondary amine, characterized in that this secondary amine, comprising at least one hydrogen atom on at least one carbon atom alpha to the nitrogen, is oxidized in the presence of an oxidizing agent and of at least one compound chosen from carbon dioxide, hydrogencarbonates and carbonates.

2. Process according to Claim 1, characterized in that the oxidizing agent is hydrogen peroxide.

3. Process according to Claim 1 or 2, characterized in that the reaction is carried out in the presence of a solvent.

4. Process according to Claim 3, characterized in that the solvent is water.

5. Process according to one of Claims 1 to 4, characterized in that the secondary amine is diisopropylamine.

6. Process according to one of Claims 1 to 5, characterized in that the temperature of the reaction mixture is between 20°C and 80°C.

7. Process according to Claim 6, characterized in that the temperature of the reaction mixture is between 50 and 70°C.

8. Process according to one of Claims 1 to 7, characterized in that the oxidizing agent/secondary amine molar ratio is between 1 and 4.

9. Process according to one of Claims 1 to 8, characterized in that the carbon dioxide and/or hydrogencarbonate and/or carbonate/secondary amine molar ratio is between 0.1 and 1.

10. Process according to Claim 9, characterized in that the carbon dioxide and/or hydrogencarbonate and/or carbonate/secondary amine molar ratio is between 0.1 and 0.3.

11. Process for obtaining N-monosubstituted hydroxylamine, characterized in that the nitrone formed according to one of Claims 1 to 10 is hydrolysed.

12. Process according to Claim 11, characterized in that the hydrolysis of the nitrone is carried out in the presence of inorganic or organic acids.

13. Process according to Claim 12, characterized in that the H⁺/secondary amine molar ratio is between 0.9 and 2.
